# EUROPEAN PATENT APPLICATION

(11) **EP 1 666 059 A1**
(43) Date of publication of application: **07.06.2006**
(21) Application number: 04771475.3
(22) Date of filing: 10.08.2004
(51) Int. Cl.: A61K 39/12, A61K 39/09, A61K 39/106, A61K 39/13, A61K 39/145, A61K 39/165, A61K 39/20, A61K 39/21, A61K 39/235, A61K 39/245, A61K 39/25, A61K 39/285, A61K 39/39, A61P 31/12

(54) **NOVEL VACCINE CONTAINING ADJUVANT CAPABLE OF INDUCING MUCOSAL IMMUNITY**

(30) Priority: 11.08.2003 JP 2003291879
(71) Applicant: The Research Foundation for Microbial Diseases of Osaka University, Suita-shi, Osaka 565-0871 (JP); JAPAN as represented by DIRECTOR GENERAL OF NATIONAL INSTITUTE OF INFECTIOUS DISEASES, Tokyo 162-8640 (JP); TORAY INDUSTRIES, INC., Tokyo 103-8666 (JP)
(72) Inventor: HASEGAWA, Hideki, Tokyo 1130033 (JP); KURATA, Takeshi, Tokyo 1850034 (JP); SATA, Tetsutarou, Tokyo 1360076 (JP); MORIYAMA, Masami, Yokohama-shi, Kanagawa 2320064 (JP); TAMURA, Shin-ichi, Osaka 5650862 (JP); TANIMOTO, Takefumi, Osaka 5650854 (JP)
(74) Representative: Woods, Geoffrey Corlett
(86) International application number: PCT/JP2004/011488
(87) International publication number: WO 2005/014038

(57) **Abstract**

The present invention provides an adjuvant that possesses a greater adjuvant potential than that of a conventional adjuvant, and that is capable of producing a protective reaction across different strains. This problem has been solved by the finding that a double-stranded RNA (for example, Poly(I:C)) unexpectedly exhibits the above capability when used in combination with a subunit antigen. Accordingly, the present invention provides a vaccine for mucosal administration containing A) a double-stranded RNA and B) a subunit antigen or inactivated antigen of a pathogen.

## Description

### Technical Field

The present invention relates to a novel vaccine composition. More specifically, the present invention relates to a novel vaccine using a double-stranded RNA as an adjuvant.

### Background Art

Currently available approved vaccines have limitations as described below. For example, in influenza viruses (particularly type A influenza virus), antigen mutations occur remarkably, resulting in the frequent emergence of viruses that are not neutralized by the antibodies produced by previously administered vaccines (i.e., already acquired infections); vaccine effect often lasts only during a single season. Also, immunologically different novel strains often emerge due to point mutations (antigenic drift) in the genes that encode surface glycoproteins (hemagglutinin [HA] and neuraminidase [NA]) and antigenic shift. Note that in this case, internal proteins are conserved at relatively high levels even within continuously mutated strains and within discontinuously mutated strains. Because immunization with currently available vaccines only induces humoral immunity among homogenous strains, rather than common immunity among heterogeneous strains based on cellular immunity, the vaccine effect will lessen if the epidemic strain and the vaccine strain differ.

Another drawback resides in that immunization must be performed every year because antibody titer decreases even if the prevailing endemic strain of influenza virus is not significantly antigenically shifted or antigenically drifted from a year to the following year. It has been reported that antibodies for hemagglutination inhibition (HI) and neutralization persist for several months to several years and then diminish gradually. However, even without such reductions, once-a-year inoculation is recommended. This is because antibody titer can decrease within the year following vaccination.

There is room for improving vaccine efficacy. This is because the development of a vaccine for the coming season depends on predicting the coming epidemic strain. Hence, this prediction is associated with inaccuracy, and a mismatch can occur between the strain used for the vaccine and the strain that is actually epidemic outdoors. Also, if a new type of strain emerges, a predicted vaccine is often ineffective, for example, against the emergence of the novel H3N2 strain (A/Beijing/92) throughout the influenza season of 1992 and 1993. A new type of virus is often not clinically evident until the late stage of influenza season, and protection with existing vaccines is often unsatisfactory because of the time required to produce and prepare an approved vaccine. Even if the vaccine strain and the epidemic strain match well with each other, approved vaccines are said to only prevent the disease in about 70% of children and adolescents and 30 to 40% of elderly persons.

With conventional vaccines, it is nearly impossible to perform mucosal (for example, nasal cavity) immunization; in particular, currently available inactivated vaccines, component vaccines and the like of the subcutaneous inoculation type, which represents the mainstream of vaccination for influenza viruses, have been known to be incapable of producing mucosal immunity; there is a strong demand for a vaccine composition capable of producing such mucosal immunity.

Also, because conventional vaccines are incapable of producing cross immunity even between different strains, there is also a strong demand for the development of a vaccine capable of producing cross immunity at least between different strains or between different subtypes. For influenza vaccines, for which the mainstream is a mixture of two kinds of type A strains and one kind of type B strain, there is a strong demand particularly for the development of a vaccine that obviates or at least reduces the need for prediction of endemic strain.

Furthermore, a vaccine effective in mucosal inoculation, which is a convenient method of vaccination, is also in much need. Since this has not been achieved for influenza viruses and the like, in particular, a resolution is awaited also for the sake of facilitating mass immunization. Alternatively, a vaccine that increases the duration of antibody is also in much need.

Non-patent document 1 (J. Clinical Investigation, 110(8), 1175-1184 (2002)) discloses that an UV-inactivated whole influenza virus with Poly(I:C) as an adjuvant was administered via the airway route. However, the fact that IgG antibody elevation does not differ between with and without the addition of Poly(I:C) is shown in Figure 2 of non-patent document 1. Hence, Poly(I:C) has been suggested to be not much effective as an adjuvant. The same document also describes a combination with a short-chain phospholipid to elevate antibody levels.

Non-patent document 2 (Invest. Ophthalmol., 10(10), 750-759 (1971)) and non-patent document 3 (Invest. Ophthalmol., 10(10), 760-769 (1971)) describe nasal inoculation with an inactivated vaccinia virus with Poly(I:C) as an adjuvant. However, non-patent document 2 describes increased IgA antibody production in tears but does not mention an infection-preventive effect.

Patent document 1 (Japanese Patent Examined Publication No. SHO-50-2009 (US3906092), Merck & Co.) discloses that antibody reactions of influenza vaccines are enhanced by adding a polynucleotide (comprising Poly(I:C)) to an adsorption type adjuvant. However, patent document 1 does not mention an infection-preventive effect.

Non-patent document 4 (Veterinary Microbiology, 88(4), 325-338 (2002)) reports on significantly elevated IgG and IgM levels after intraperitoneal inoculation of an inactivated vaccine with Poly(I:C) as an adjuvant, but does not show an effect by mucosal administration to the nasal cavity and the like and, in addition, does not mention an infection-preventive effect.

Non-patent document 5 (Proc. Soc. Exp. Biol. & Med., 133, 334-338 (1970)) reports that blood antibody levels rise when sheep erythrocytes with Poly(I:C) as an adjuvant are injected intravenously for immunization, but does not mention an infection-preventive effect.

Non-patent document 6 (The Journal of Immunology, 149, 981-988 (1992)) describes a potential of cholera toxin as an adjuvant, but does not describe a double-stranded RNA at all.
Patent document 1: Japanese Patent Examined Publication No. SHO-50-2009
Non-patent document 1: J. Clinical Investigation, 110 (8), 1175-1184 (2002)
Non-patent document 2: Invest., Ophthalmol., 10(10), 750-759 (1971))
Non-patent document 3: Invest., Ophthalmol., 10(10), 760-769 (1971)
Non-patent document 4: Veterinary Microbiology, 88(4), 325-338 (2002)
Non-patent document 5: Proc. Soc. Exp. Biol. & Med., 133, 334-338 (1970)
Non-patent document 6: The Journal of Immunology, 149, 981-988 (1992)

### Summary of the Invention

### Problem to Be Solved by the Invention

In the circumstances described above, the present invention is intended to provide an adjuvant that exhibits an adjuvant potential greater than that of any conventional adjuvant and is capable of producing protective reactions across different strains when given by mucosal administration.

### Means of Solving the Problem

The above-described problem has been solved by the finding that a double-stranded RNA (for example, Poly(I:C)) unexpectedly exhibits the above-described potential when used in combination with a subunit antigen.

Although currently available inactivated influenza HA vaccines are effective vaccines as described above, IgA antibody induction in the respiratory mucosal epithelium, which is the gait for entry of influenza viruses, is of low extent; therefore, improving this induction is considered to further enhance the effect.

With this in mind, the present inventors attempted to produce a secretory IgA antibody showing high cross reactivity to the airway mucosa using a nasal vaccine in combination with an adjuvant in a mouse model of influenza. Good results were obtained when a cholera toxin B subunit (CTB*) was used as an adjuvant. Considering nasal administration in humans, however, cholera toxin has been reported to cause adverse effects such as facial nerve paralysis; therefore, it is thought that the vaccine will become safer, provided that IgG antibody can be induced without using an adjuvant not comprising cholera toxin. IgA activates the second pathway of the complement system and plays an important part in local immune reactions in mucosal infections; because IgA as such is metabolized with a plasma half-life of 5 to 6 days, it is thought to be impossible to predict an infection-preventive effect, for example, even if IgA in tears increases.

Hence, the present inventors investigated to determine whether a ligand for the Toll-like receptor (TLR), which recognizes microbial components in the body and stimulates the innate immunity system, has the potential for a highly safe, strong adjuvant for mucosal vaccines, and obtained a vaccine for mucosal administration having an unexpected infection-protective effect.
Accordingly, the present invention provides the following:
(1) A vaccine for mucosal administration comprising:
   A) a double-stranded RNA; and
   B) a subunit antigen or inactivated antigen of a pathogen.
(2) The vaccine of (1), wherein the above-described mucosa comprises the nasal mucosa.
(3) The vaccine of (1), wherein the above-described pathogen is selected from the group consisting of varicella virus, measles virus, mumps virus, poliovirus, rotavirus, influenza virus, adenovirus, herpes virus, rubella virus, severe acute respiratory syndrome virus (SARS virus), human immunodeficiency virus (HIV), Bordetella *pertussis, Neisseria* meningitidis, *Haemophilus influenzae* type b, *Streptococcus pneumoniae, and Vibrio cholerae.*
(4) The vaccine of (1), wherein the above-described pathogen is an influenza virus.
(5) The vaccine of (1), wherein the above-described subunit comprises at least one subunit selected from the group consisting of the influenza virus subunits HA, NA, M1, M2, NP, PB1, PB2, PA and NS2.
(6) The vaccine of (1), wherein the above-described double-stranded RNA is present at a concentration sufficient to produce secretory IgA.
(7) The vaccine of (1), wherein the above-described double-stranded RNA is present at a concentration of 0.1 to 10 mg/ml.
(8) The vaccine of (1), wherein the size of the above-described double-stranded RNA is 10² to 10⁸ bp.
(9) The vaccine of (1), wherein the above-described subunit comprises at least NA or HA.
(10) The vaccine of (1), wherein the above-described double-stranded RNA comprises Poly(I:C).
(11) A method of preventing an infectious disease, comprising:
   a step for mucosally administering at least once:
      A) a vaccine for mucosal administration comprising:
         a) a double-stranded RNA; and
         b) a subunit antigen or inactivated antigen of a pathogen.
(12) The method of (11), wherein the above-described vaccine is administered at least twice.
(13) The method of (11), wherein the above-described vaccine is administered at an interval of at least 1 week or more, more preferably 3 weeks or more.
(14) The method of (11), wherein the above-described double-stranded RNA comprises Poly(I:C).
(15) A vaccine kit for preventing an infectious disease, provided with:
   A) a vaccine for mucosal administration comprising:
      a) a double-stranded RNA; and
      b) a subunit antigen or inactivated antigen of a pathogen; and
   B) an instruction sheet directing to mucosally administer the above-described vaccine at least once.
(16) The kit of (15), wherein the above-described vaccine is administered at least twice.
(17) The kit of (15), wherein the above-described vaccine is administered at an interval of at least 1 week or more, more preferably 3 weeks or more.
(18) The kit of (15), wherein the above-described double-stranded RNA comprises Poly(I:C).
(19) A use of a double-stranded RNA for mucosal administration of a vaccine.
(20) The use of (19), wherein the above-described double-stranded RNA comprises Poly(I:C).
(21) A use of a double-stranded RNA for production of a vaccine for mucosal administration.
(22) The use of (21), wherein the above-described double-stranded RNA comprises Poly(I:C).

### Effect of the Invention

The present invention provides a form of vaccine that enables easy vaccination by mucosal administration and obtainment of cross immunity. In the case of influenza viruses, for example, it is thereby possible to produce an effective vaccine without predicting the epidemic strain, and hence to take efficient prophylactic measures.

### Brief Description of the Drawings

Fig. 1 presents data showing the adjuvant effect of Poly(I:C) as exemplified in Examples. The left panel shows dosage forms; the middle panel shows IgA contents in nasal washings; the right panel shows IgA contents in serum.
Fig. 2 presents data showing the adjuvant effect of Poly(I:C) as exemplified in Examples. The left panel shows dosage forms; the right panel shows virus survival status.
Fig. 3 is a drawing showing the IgA-producing effects of the vaccination of the present invention on various viral strains.
Fig. 4 is a drawing showing suppressive effects on the viral growth of the vaccination of the present invention on various viral strains.
Fig. 5 is a drawing showing the toxicity of the vaccination of the present invention in intracerebral administration. The upper panel shows data for the Poly(I:C) of the present invention; the lower panel shows data for the positive control CTB*.
Fig. 6 shows the immune effects of inactivated virus particles used as a nasal influenza vaccine in combination with Poly(I:C), i.e., anti-HA and anti-NA antibody titers in nasal washings and serum.
Fig. 7 shows the immune effects of inactivated virus particles used as a nasal influenza vaccine in combination with various sizes (L, M, H) of Poly(I:C), i.e., anti-HA and anti-NA antibody titers in nasal washings and serum.
Fig. 8 shows the immune effects of a double-stranded RNA or single-stranded RNA used as a nasal influenza vaccine in combination with the subunit HA, i.e., anti-HA antibody titers in nasal washings and serum.

Fig. 9 presents results showing the efficacy of a nasal influenza vaccine in combination with Poly (I:C) administered twice or more at an interval of 1 week or more.

Fig. 10 presents results showing that Poly(I:C) also increases protective immunity for a pertussis vaccine, and is also effective in enhancing protective immunity for vaccines for non-influenza infections.

### Best Modes for Embodying the Invention

The present invention is hereinafter described in more detail. Throughout the entire description, any expression in singular form is to be understood to encompass the plural form thereof unless otherwise stated. Additionally, the terms as used herein are to be understood to be used with meanings commonly used in the art unless otherwise stated.

### (Definitions)

The term "vaccine" as used herein refers to an antigenic suspension or solution usually comprising an infectious factor or a portion of an infectious factor, administered into the body to produce active immunity. The antigenic portion that constitutes a vaccine can be a microorganism (for example, virus or bacterium and the like) or a natural product purified from a microorganism, a synthetic or genetically engineered protein, peptide, polysaccharide or similar product. Examples of live vaccines include, but are not limited to, BCG, smallpox vaccination, polio, varicella, measles, rubella, mumps, rinderpest, NDV, Marek's disease and the like. Inactivated vaccines include, but are not limited to, pertussis, diphtheria (toxoid), tetanus (toxoid), influenza, Japanese encephalitis and the like.

The term "inactivated antigen" as used herein refers to an antigen deprived of infectivity, used as a vaccine antigen; such antigens include, but are not limited to, complete virus particle virions, incomplete virus particles, virion-constituting particles, virus non-structural proteins, antigens that protect against infections, neutralizing reaction epitopes and the like. The term "inactivated antigen" as used herein refers to an antigen deprived of infectivity, but retaining immunogenicity; when such an antigen is used as a vaccine, it is called an "inactivated vaccine." Examples of such inactivated antigens include, but are not limited to, those inactivated by physical (for example, X-ray irradiation, heat, ultrasound), chemical (formalin, mercury, alcohol, chlorine) or other procedures. Subunit antigen *per* se also falls within the definition of inactivated antigen because they have usually lost infectivity. Alternatively, a killed virus may be used.

The term "subunit antigen" of a virus as used herein is also called "component"; such a subunit antigen may be purified from a pathogen such as a naturally occurring virus, or may be prepared by a synthetic or recombinant technology. Such methods are well known and in common use in the art, and can be performed using commercially available equipment, reagents, vectors and the like. For example, in the case of influenza viruses, the subunit antigen is preferably a molecule exposed to the surface of the particle, such as hemagglutinin (HA), neuraminidase (NA), matrices (Ml, M2), non-structures (NS), polymerases (PB1, PB2: basic polymerases 1 and 2, acidic polymerase (PA)), and nuclear proteins (NP). Currently, HA is known to occur in 15 kinds, and NA in 9 kinds; a change in the kind thereof can produce a new strain.

The term "adjuvant" as used herein refers to a substance that increases or otherwise alters immune responses when mixed with an administered immunogen.

The term "CT" or "cholera toxin" as used herein refers to an exotoxin produced by *Vibrio cholerae,* which is a causal substance for diarrheal symptoms due to *Vibrio cholerae* infection. Although cholera toxin is used as an effective adjuvant, it has not found a clinical application because of its toxicity. Therefore, CT is usually used as a positive control when searching for an effective adjuvant for a vaccine.

The term "double-stranded RNA" as used herein refers to an optionally chosen double-stranded RNA. The size thereof can be measured by, for example, gel electrophoresis and the like. Traditionally, attempts have been made to use a double-stranded RNA as an adjuvant for a vaccine, but there have been almost no reports that a vaccine was effective in protecting against infections. Such double-stranded RNAs include, but are not limited to, Poly(I:C), Poly(A:U), Poly(G:C) and the like.

The term "Poly(I:C)" as used herein refers to a double-stranded RNA comprising polyinosinic acid (pI) and polycitidic acid (pc), and falls within the above-described scope of double-stranded RNA.

In the present description, any of an inactivated antigen and a subunit antigen can be used as the antigen.

The term "mucosal administration" as used herein refers to a dosage form given via the mucosa. The term "mucosa" as used herein refers to the inner wall of a hollow organ, particularly an organ that communicates with the outside of the body, such as a gastrointestinal organ, a respiratory organ, or a urogenital organ, in a vertebral animal. Accordingly, examples of such routes of mucosal administration include, but are not limited to, nasal cavity administration (nasal administration), buccal administration, intravaginal administration, upper airway administration, alveolar administration and the like. Preferably, nasal cavity administration is advantageous. This is because the nasal cavity is also a route of infection in respiratory infectious diseases, particularly influenza viruses, and hence can cause IgA reactions by mucosal administration.

The term "nasal administration" as used herein refers to a method of administration via the nasal mucosa.

The term "pathogen" as used herein refers to an organism capable of producing a disease or disorder in a host. Examples of pathogens for humans include, but are not limited to, viruses, bacteria, protozoa, rickettsia, chlamydia, fungi and the like. Pathogens against which vaccines are effective usually include, but are not limited to, viruses, bacteria and the like.

The virus targeted herein may be of any kind, and includes, but is not limited to, DNA viruses, RNA viruses and the like.

Examples of viruses that are pathogens to humans include, but are not limited to, varicella virus, measles virus, mumps virus, poliovirus, rotavirus, influenza virus, adenovirus, herpes virus, rubella virus, SARS virus (a kind of coronavirus), and HIV. The virus is preferably an influenza virus.

The bacterium targeted in the present invention may be any bacterium, and includes, but is not limited to, Gram-positive bacteria and Gram-negative bacteria.

Bacteria that are pathogens to humans include, but are not limited to, *Bordetella pertussis, Neisseria meningitidis, Xaemophilus influenzae* type b, *Streptococcus pneumoniae, Vibrio cholerae* and the like.

The term "influenza virus" as used herein refers to a single-stranded RNA virus belonging to the family *Orthomyxoviridae.* The virus has an envelop of lipid double membrane, backed by M1 (membrane protein), in which membrane characteristic membrane proteins such as M2, HA (hemagglutinin), NA (neuraminidase) and M2 glycoprotein are embedded. The RNA occurs in eight segments, which, along with nuclear proteins, have formed a complex RNP (ribonucleoside capsid) and are weakly bound to the envelop-backing protein M1.

Of the influenza virus proteins, HA and NA are produced as embedded in the endoplasmic reticulum membrane, and are exposed to the cell surface via the Golgi apparatus. Therefore, HA or NA or both are good immunogens, and are used as major starting materials for vaccines.

The term "concentration sufficient to produce secretory IgA" as used herein refers to an ability of an adjuvant or a vaccine *per se,* i.e., a concentration of the adjuvant or the vaccine per se that allows production of secretory IgA upon onset of an immune reaction after administration. Such a concentration can be achieved in vitro or in vivo using a method publicly known in the art.

The term "secretory IgA" as used herein refers to an IgA that is secreted. IgA is a major immunoglobulin in exocrine fluids, and is helpful in protection against infections on the mucosal surface. Although this IgA is abundantly found in saliva, nasal discharge, and fluids secreted from the intestine, trachea and the like, or in colostrum, it is also present in serum. As such, secretory IgA can be measured by, for example, immunodiffusion, which, however, is not to be construed as limiting; as examples of preferably usable methods, those described in Examples can be mentioned.

### (Description of general biochemical techniques that can be used in the present invention)

### (Method of preparing a vaccine)

In the present description, a subunit antigen or inactivated antigen contained in a vaccine can be prepared from a natural material by inactivation, purification and the like, as described above, or can be artificially prepared by preparing a polypeptide by genetic engineering technology or by synthesis. Usually, the vaccine of the present invention can be produced by growing a virus and the like using a developed egg and the like, and inactivating the grown virus and the like or separating and purifying a component therefrom.

In the present description, the vaccine of the present invention can be supplied in a liquid or dried form in a tightly stoppered vial, syringe, atomizer or the like, or in a thermally sealed ampoule.

When an influenza virus vaccine is produced, the following procedures can be used, but are not to be construed as limiting.

Examples of the desired influenza virus strain include, but are not limited to, A/Beijing/352/89(H3N2); A/Texas/36/91(H1N1); B/Panama/45/90; A/Georgia/03/93; A/New Caledonia/20/99(H1N1), A/Panama/2007/99(H3N2); B/Shangdong/7/97; B/Johannesburg/5/99 and the like.

These viruses are, for example, grown by passage incubation in 9- to 11-day developed embryos of eggs and, if necessary, grown in cultured cells (for example, MDCK cells). Viruses can be purified by the method described by Massicot et al. (Virology 101, 242-249 (1980)) or a modification thereof. Briefly, a virus suspension is clarified by centrifugation at 8000 rpm (for example, Sorvall RC5C centrifuge, GS-3 rotor), then pelletized by centrifugation using a Beckman 19 model rotor at 18,000 rpm for 2 hours.

The pelletized virus is resuspended in STE (0.1M NaCl, 20mM Tris, pH 7.4, 1mM EDTA) and centrifuged at 4,000 rpm for 10 minutes (Hermle Z360K centrifuge), and the aggregate is removed. 2 ml of the supernatant is overlaid on a discontinuous sucrose gradient consisting of 2 ml of 60% sucrose and 7 ml of upper STE-buffered 30% sucrose, and centrifuged at 36,000 rpm (SW-40 rotor, Beckman) for 90 minutes.

The banded virus is collected at the interface, diluted 10 fold with STE, and pelletized at 30,000 rpm for 2 hours (Beckman Ti45 rotor). Subsequently, the pelletized virus is frozen at -70°C.

A subunit antigen of a virus can be produced by cultivation (for example, CHO-K1 cells) using recombinant DNA technology. Expression vectors that can be used include, but are not limited to, pCXN (Matsunami K. et al., (Clinical & Experimental Immunology 126(1), 165-172 (2001)) and the like. Transformed cells are dissolved in a solubilizing buffer solution (8% Triton X-100, 2M KC1, 10mM sodium phosphate buffer (pH 7.0)) or the like, and suspended by the addition of an equal volume of PBS, followed by, for example, centrifugation at 360,000 rpm (for example, Beckman XL-70 centrifuge Type 55.1Ti rotor), whereby a soluble fraction is recovered. The recovered soluble fraction can be adsorbed to an affinity column wherein a protein, a peptide or the like, such as a monoclonal antibody or polyclonal antibody, possessing specific affinity for the desired antigen or a peptide sequence added thereto, is coupled to a carrier, and eluted and purified using a solution that weakens the binding force due to a pH change or another change, such as 0.1M glycine-HCl or 0.1% Tween 80 (pH 2.7). Also, techniques such as solvent extraction, salting-out desalinization by ammonium sulfate precipitation, precipitation with an organic solvent, anion exchange chromatography using a resin such as diethylaminoethyl (DEAE)-Sepharose or DIAION HPA-75 (Mitsubishi Chemical Corporation), hydrophobicity chromatography using a resin such as butyl-Sepharose or phenyl-Sepharose, gel filtration using a molecular sieve, chromatofocusing, and isoelectric focusing, can also be used. The purified antigen is dialyzed against a buffer solution such as PBS, and can be frozen at, for example, -70°C.

In these forms, a vaccine can be produced.

### (Adjuvant)

The term adjuvant generically refers to substances that increase antibody production and enhance immune responses when combined with an antigen; in a more preferred mode of embodiment, a moduolatory or effective, non-toxic adjuvant is used. Adjuvants are required to be used along with an ordinary vaccine antigen to induce quicker, more potent, or prolonged responses. As such, adjuvants are also useful in cases where antigen supply is limited, or antigen production is costly.

Adjuvants are classified into, for example, minerals, bacteria, plants, synthetic products, or host products.

Adjuvants of the first class are mineral adjuvants, for example, aluminum compounds. The first use of an aluminum compound as an adjuvant was described in 1926. Since then, antigens co-precipitated with an aluminum compound or antigens mixed with, or adsorbed to, a previously formed aluminum compound, have been used to enhance immune responses in animals and humans. Aluminum compounds and similar adjuvants seem to act by the mechanism described below. Aluminum physically binds to an antigen to form particles and slows the rate of the absorption of the antigen in tissue after injection, thus extending the period of interaction between the antigen and antigen-presenting cells, for example, macrophages or follicular-dendritic cells. Alternatively, adjuvants further activate such interactions. Aluminum particles are demonstrated to appear locally in rabbit lymph nodes at 7 days after immunization, and can direct the antigen to a T-cell-containing region in the lymph nodes themselves by other significant function. Adjuvant potency has been shown to bear a correlation with activation of relevant lymph node. Although a large number of studies have demonstrated that an antigen administered along with aluminum activates humoral immunity, cellular immunity seems to increase only slightly. Aluminum has also been described as activating the routes of complements. This mechanism can play a role in local inflammatory reactions and immunoglobulin memory.

Aluminum compounds are nearly the only safe adjuvant that is currently used in humans. However, aluminum-containing vaccines sometimes cause local reactions. Although the onset of allergies is usually of no major clinical concern, aluminum compounds reportedly mobilize eosinophils to injection sites via a T-cell-dependent mechanism, to induce IgE responses after antigen priming, and to activate a population of specific cells having helper function for IgE responses.

Adjuvants of bacterial origin have recently been purified and synthesized (for example, muranyldipeptide, lipid A). Also, host-derived immunologically active proteins have been cloned (interleukin 1 and interleukin 2). In recent years, *Bordetella pertussis,* lipopolysaccharides and Freund's complete adjuvant (FCA) have become used at laboratory levels.

Other various substances have also become used as adjuvants. These include plant products, for example, saponin, animal products, for example, chitin, and a large number of synthetic chemical substances.

In the present description, a double-stranded RNA is used as an adjuvant. This double-stranded RNA can be prepared in accordance with the above-described method of preparing a nucleic acid molecule, and a method well known in the art can be used. As examples of such methods, kits available from Sigma Aldrich Japan, YAMASA Corporation, Fluka and elsewhere can be used.

Poly(I:C) can also be produced using a method well known in the art. Such methods are described in non-patent documents 1 to 3 and the like, and examples of preferable methods include, but are not limited to, mixing two selected homopolymers in a phosphate-buffered solution at pH 7.0 (0.006 mol sodium phosphate, 0.15 mol sodium chloride) at an equimolar concentration and the like. The complex can be formed immediately after mixing.

### (Computer screening)

For protein conformation data screening, a factor (for example, antigen or inactivated antigen, antibody), polypeptide or nucleic acid molecule of the present invention can be used. The screening may be performed using an in vitro, in vivo or other system using an existing substance, or using a library produced using an in silico screening (computer-based system) system. In the present invention, it is understood that compounds of desired activity obtained by screening are also encompassed in the scope of the present invention. In the present invention, it is also intended that a drug designed by computer modeling is provided on the basis of the disclosure of the present invention. Accordingly, a drug obtained by such screening can also be used as a component for the vaccine of the present invention.

### (Diseases)

Diseases that can be targeted by the present invention in the present description include optionally chosen diseases that can be prevented by vaccine administration. Such diseases include, but are not limited to, bacterial diseases, viral diseases, allergic diseases and the like; examples include, but are not limited to, varicella, measles, mumps, polio, rota, influenza, rubella, severe acute respiratory syndrome (SARS), pertussis, meningitis and cholera, RS (respiratory syncytium) viral infection, *Haemophilus influenzae* type b, *Streptococcus pneumoniae* infections, acquired immunodeficiency syndrome (AIDS) and the like.

### (Demonstration of therapeutic activity or prophylactic activity)

The compound or pharmaceutical composition of the present invention is preferably tested for desired therapeutic activity or prophylactic activity in vitro, then in vivo, and at animal levels, prior to use in humans. The effects of the compound or composition on a cell strain and/or tissue sample can be determined using a technique known to those skilled in the art. As in vitro assays that can be used to determine whether administration of a particular compound is indicated, according to the present invention, observation for antigen-antibody binding and the like can be mentioned. In animal-level testing, the judgment can be made by administering the test vaccine as in humans, and confirming an increase in antibody titer (determined by, for example, ELISA), or cytotoxic T cell activation and the like.

### (Administration and composition for prophylaxis)

Pharmaceutically acceptable carriers that can be used in the composition, vaccine and the like of the present invention include, but are not limited to, antioxidants, preservatives, colorants, flavoring agents, and diluents, emulsifiers, suspending agents, solvents, fillers, bulking agents, buffering agents, delivery vehicles, diluents, excipients and/or pharmaceutical adjuvants. Typically, the pharmaceutical of the present invention is administered in the form of a composition comprising a vaccine or a modification or derivative thereof along with one or more physiologically acceptable carriers, excipients or diluents. For example, the appropriate vehicle can be water for injection, a physiological solution, or an artificial cerebrospinal fluid, and these can be supplemented with other substances commonly used in compositions for non-oral delivery.

The acceptable carrier, excipient or stabilizer used herein is not-toxic to the recipient, and is preferably inert at the dose and concentration used; examples include, but are not limited to, phosphates, citrates, or other organic acids; ascorbic acid, α-tocopherol; low-molecular-weight polypeptides; proteins (for example, serum albumin, gelatin or immunoglobulin); hydrophilic polymers (for example, polyvinylpyrrolidone); amino acids (for example, glycine, glutamine, asparagine, arginine or lysine); monosaccharides, disaccharides and other carbohydrates (including glucose, mannose, or dextrin); chelating agents (for example, EDTA); sugar alcohols (for example, mannitol or sorbitol); salt-forming counterions (for example, sodium); and/or non-ionic surfactants (for example, Tween, pluronic or polyethylene glycol (PEG)) and the like.

As examples of the appropriate carrier, neutrally buffered physiological saline, or physiological saline admixed with serum albumin can be mentioned. Preferably, the product is formulated as a lyophilized product using an appropriate excipient (for example, sucrose). Other standard carriers, diluents and excipients can be contained as desired. Other representative compositions comprise a Tris buffer at pH 7.0-8.5 or an acetate buffer at pH 4.0-5.5, and these can further comprise sorbitol or an appropriate alternative thereto.

A general method of preparing the pharmaceutical composition of the present invention is described below. Note that animal drug compositions, quasi drugs, aquaculture drug compositions, food compositions and cosmetic compositions and the like can also be produced by publicly known methods of preparation.

The vaccine and the like of the present invention can be administered non-orally as blended with a pharmaceutically acceptable carrier.

The pharmaceutical of the present invention can be prepared and preserved in the form of a lyophilized cake or aqueous solution by mixing as necessary a physiologically acceptable carrier, excipient or stabilizer (see Japanese Pharmacopoeia XIV or the current update thereof, Remington's Pharmaceutical Sciences, 18th Edition, A.R. Gennaro, ed., Mack Publishing Company, 1990 and the like) and a sugar chain composition of desired level of purity.

Various drug delivery systems are publicly known, and in the present invention, mucosal administration is intended. As examples of techniques used to administer the compound of the present invention, liposomes, microparticles, microcapsules and the like can be mentioned. Methods of introduction thereof include, but are not limited to, mucosal routes such as nasal cavity, intravaginal, sub-airway, buccal, rectal mucosal and intestinal mucosal routes. In this case, the compound of the present invention can be administered along with another biologically active drug. The administration can be systemic or topical. In the case of mucosal administration, pulmonary administration can also be used by, for example, using an inhalator or sprayer, and a formulation using an aerosol agent.

In a particular mode of embodiment, it is desirable that the compound or composition of the present invention be administered topically not only to the mucosal surface at the administration site but also to the mucosal surface of another tissue wherein IgA secretion can be increased.

The amount of composition used in the prophylactic method of the present invention can easily be determined by those skilled in the art in view of the purpose of use, target disease (kind and the like), the patient's age, body weight, history of illness and the like. Frequency of application of the method of treatment of the present invention to a subject (or patient) can also easily be determined by those skilled in the art in view of the purpose of use, target disease (kind, seriousness and the like), the patient's age, body weight, history of illness, course and the like. As examples of frequencies, daily to every-several-months administration (for example, weekly to monthly), or once before every epidemic season and the like can be mentioned. It is preferable that weekly to monthly administration be performed while monitoring the course, and it is advantageous to make a booster immunization at an interval of at least about 1 week. More preferably, the interval to the booster immunization can be at least about 3 weeks.

Although the dose of the vaccine and the like of the present invention varies depending on the subject's age, body weight, symptoms or method of administration and the like, and is not subject to limitation, it can normally be 10 mg to 1 g per day for oral administration in an adult. In the case of mucosal (for example, nasal) administration, the dose is 0.001 mg to 10 mg, and can preferably be 0.1 mg to 1 mg.

The term "administer" as used herein means that the vaccine and the like of the present invention or a pharmaceutical composition containing the same is given to a host to be treated, alone or in combination with another therapeutic agent. The combination can be administered, for example, simultaneously in a mixture, separately but simultaneously or concurrently; or sequentially. This includes presentation wherein the combined drugs are administered together in a therapeutic mixture, and also includes procedures wherein the combined drugs are administered separately but simultaneously (for example, to the same individual via separate mucosae). "Combination" administration further includes separately administering one of the compounds or drugs given first and subsequently given second.

The term "instruction sheet" as used herein refers to a document bearing information on how to administer the pharmaceutical and the like of the present invention or how to make a diagnosis and the like for a person who performs administration, such as a physician or patient, and a person who makes a diagnosis (can be the patient). This instruction sheet bears a statement of directions concerning the procedures for administering the diagnostic agent, prophylactic drug, pharmaceutical and the like of the present invention. This instruction sheet is prepared in accordance with a form specified by the supervising authorities of the country in which the present invention is embodied (for example, Ministry of Health, Labor and Welfare for Japan, Food and Drug Administration (FDA) for the United States and the like), and states that approval has been obtained from the supervising authorities. The instruction sheet is what is called a package insert, and is usually supplied in a paper medium, which, however, is not to be construed as limiting; the instruction sheet can also be supplied in the form of, for example, a film applied to a bottle, or an electronic medium (for example, home pages (websites) provided via the Internet, e-mails).

A judgment on completion of a prophylactic treatment by the method of the present invention can be made by confirming an elicited antibody using a commercially available assay or equipment.

The present invention also provides a pharmaceutical package or kit having one or more vessel containing one or more component of the pharmaceutical composition of the present invention. Such vessels can have an optionally attached notice in a form specified by the governmental organization that regulates the manufacture, use or sales of pharmaceutical products or biological products, which notice indicates approval for the manufacture, use or sales for administration in humans by the governmental organization.

### (Description of preferred modes of embodiment)

Preferred modes of embodiment of the present invention are hereinafter described. The modes of embodiment given below are described for the sake of better understanding of the invention, and the scope of the present invention is understood not to be limited to the following description. It is evident, therefore, that those skilled in the art are able to modify any mode of embodiment as appropriate within the scope of the present invention, in consideration of the description herein.

In one aspect, the present invention provides a vaccine for mucosal administration. This vaccine comprises A) a double-stranded RNA; and B) a subunit antigen or inactivated antigen of a virus. Here, the double-stranded RNA and viral subunit antigen and inactivated antigen can be prepared by methods commonly known in the art. Appropriate forms for mucosal administration are well known in the art, and examples include, but are not limited to, liquids, or sprays and the like. The present invention has been demonstrated to raise the secretory IgA titer of the airway mucosa and actually have an infection-protective effect by combining a double-stranded RNA and a viral subunit antigen or inactivated antigen. This effect can be deemed an unexpectedly remarkable effect, taking into account the fact that there have been a large number of reports that a double-stranded RNA, as an adjuvant, produces an antibody, but does not have an actual infection-protective effect.

Because the vaccine of the present invention accomplishes its remarkable effect by mucosal administration, any route can be followed, as long as its administration is via the mucosa (for example, nasal administration, buccal administration and the like); however, in a preferred mode of embodiment, the nasal route can be followed.

In a preferred mode of embodiment, the pathogen targeted by the vaccine of the present invention can, for example, be one selected from the group consisting of varicella virus, measles virus, mumps virus, poliovirus, rotavirus, influenza virus, adenovirus, herpes virus, rubella virus, SARS virus, HIV, Bordetella *pertussis, Neisseria meningitidis, Haemophilus influenzae type b, Streptococcus pneumoniae* and *Vibrio cholerae.* Preferably, the pathogen is an influenza virus. The present invention has an excellent effect of presenting a vaccine that exhibited cross reactivity among subtypes within a strain (for example, H1N1 and the like) within a type of influenza virus (type A, type B) substantially for the first time in history. Because the present invention exhibits cross reactivity beyond the barrier of types in some cases, the present invention has an effect that has not been accomplished by the prior art. Since the epidemic pattern of influenza virus changes every year, with changes in the virus itself, it has been conventional practice to predict the likely epidemic pattern and prepare the appropriate vaccine for influenza virus every year. However, the present invention accomplishes cross reactivity across different strains and subspecies, and thus has an effect of enabling the provision of an effective influenza vaccine without predicting the epidemic pattern. Additionally, the obviation of the need for prediction makes it possible to use long-preserved vaccines.

In a preferred mode of embodiment, the pathogen subunit used in the present invention comprises a subunit selected from the group consisting of the influenza virus subunits HA, NA, M1, M2, NP, PB1, PB2, PA and NS2. More preferably, a surface-presented subunit (for example, HA, NA) is used. More preferably, it is advantageous to use a plurality (for example, HA and NA) of this surface-presented subunit. This is because using a surface-presented subunit makes it possible to induce more effective antigen-antibody reactions and hence to induce a neutralizing antibody.

Preferably, the double-stranded RNA is present at a concentration sufficient to produce secretory IgA. Such double-stranded RNA concentrations are, for example, 0.1 to 10 mg/ml, more preferably 0.5 to 2 mg/ml, and still more preferably about 1 mg/ml (for example, 0.8 to 1.2 mg/ml).

Preferably, the double-stranded RNA is supplied in a size sufficient to produce secretory IgA. Examples of such sizes include 10² bp or more, with preference given to sizes of 0 to 3x10⁶ bp, more preferably 300 bp or more, which sizes, however, are not to be construed as limiting. The upper limit of the size of the double-stranded RNA of the present invention is not subject to limitation; examples of the upper limit of size include, but are not limited to, 10⁸ bp.

In a preferred mode of embodiment, it is advantageous that the subunit used in the vaccine of the present invention comprises at least NA or HA. This is because comprising one of these, more preferably both, makes it possible to efficiently induce a neutralizing antibody, and hence to accomplish an antiviral effect.

Although the double-stranded RNA preferably comprises Poly(I:C), other double-stranded RNAs (for example, Poly(A:U), Poly(G:C)), mixtures thereof and the like can be used. Poly(I:C) may be of any type, whether the nucleotide is altered or not.

In another aspect, the present invention provides a method of preventing an infectious disease. This method comprises a step for mucosally administering at least once A) a vaccine for mucosal administration comprising a) a double-stranded RNA (preferably Poly(I:C)); and b) a subunit antigen of a virus. Mucosal administration of the vaccine can be performed in an appropriate form according to the site of administration. In the case of nasal administration, various methods such as spraying, coating, or direct dripping of a vaccine liquid can be used.

Vaccine administration is effective preferably when performed at least twice. This way of immunization is sometimes called booster immunization. Performing booster immunization makes it possible to obtain a higher infection-protective effect.

When vaccine administration is performed a plurality of times, it is preferable that the interval be at least 1 week or more, more preferably 3 weeks or more. Modes of double-stranded RNA, Poly(I:C), antigen and the like can be performed as herein described above.

In another aspect, the present invention provides a vaccine kit for preventing an infectious disease. This kit is provided with A) a vaccine for mucosal administration comprising a) a double-stranded RNA; and b) a subunit antigen of a virus; and B) an instruction sheet directing to administer the vaccine at least once. This kit can be sold as a pharmaceutical in a package. The instruction sheet bears a statement of approval from regulatory authorities such as the Ministry of Health, Labor and Welfare and a statement indicating how to use the kit. The methods of prepare and administer the vaccine are the same as those herein described above.

### (Polypeptide form of influenza vaccine subunit antigen)

In one aspect, the present invention provides a composition for prophylaxis, treatment or prognosis for a disease, disorder or condition in an infectious disease, comprising a prophylactically, therapeutically or prognostically effective amount of an influenza vaccine subunit antigen, or a fragment or modification thereof, and a double-stranded RNA. Here, the prophylactically, therapeutically or prognostically effective amount can be determined using a technique well known in the art by those skilled in the art, in view of various parameters; such an amount can easily be determined by those skilled in the art in view of, for example, the purpose of use, target disease (kind, seriousness and the like), the patient's age, body weight, history of illness and the like (see, for example, "Vaccine Handbook", edited by the Researcher's Associates (Gaku-yuu-kai) of The National Institute of Health (1994); "Manual of Prophylactic Inoculation, 8th edition", edited by Mikio Kimura, Munehiro Hirayama, and Harumi Sakai, Kindai Shuppan (2000); "Minimum Requirements for Biological Products", edited by the Association of Biologicals Manufacturers of Japan (1993) and the like)).

The present invention is hereinafter described with reference to the following Examples, which Examples are given solely for the purpose of exemplification. Accordingly, the scope of the present invention is not limited to the Examples only, but limited only by the Scope of Claims.

### Examples

In the following Examples, experiments were performed after all subject patients provided informed consent. Animals were handled in compliance with the standards established by the National Institute of Infectious Diseases and Osaka University. The reagents used in the Examples below were obtained from either Sigma Aldrich Japan, YAMASA Corporation, or Fluka. (Example 1. Adjuvant action of Poly(I:C), a synthetic double-stranded RNA)

In this Example, the neutralizing-antibody-inducing potential and hence infection-protective effect of an inactivated virus or subunit antigen was verified using Poly(I:C), a synthetic double-stranded RNA, as an adjuvant.

### (Materials)

Mice: BALB/c mice (6 weeks of age, female)
Virus: Influenza virus H1N1 (A/PR8) strain (obtained from the National Institute of Infectious Diseases (1-23-1, Toyama, Shinjyuku-ku, Tokyo))
Vaccines: Influenza virus H1N1 (A/PR8) strain and H1N1 (A/Beijing) strain (National Institute of Infectious Diseases); H1N1 (A/Yamagata) strain (National Institute of Infectious Diseases); H3N2 (A/Guizhou) strain (National Institute of Infectious Diseases); ether-inactivated HA vaccine (Research Foundation for Microbial Diseases of Osaka University, 2-9-41, Yahatacho, Kan-onji, Kagawa Prefecture)
Adjuvants: CTB* (CTB (cholera toxin B subunit) containing 0.1% CT (cholera toxin) as a positive control,
Poly (I:C).

### (Method)

Five 6-week-old BALB/c mice per group (Japan SLC, Inc., Tokyo) were used. Five microliters (5 µl) of a mixture of 1 µg of each PR8HA vaccine (National Institute of Infectious Diseases; Research Foundation for Microbial Diseases of Osaka University) and 0.1 µg, 1 µg, 3 µg, or 10 µg of Poly(I:C) as an adjuvant for each vaccine, was inoculated to the nasal cavity of each animal; three weeks later, an equal amount of vaccine, with or without an adjuvant, was inoculated nasally; two weeks later, 1.2 µl of 100 pfu of the PR8 influenza virus was inoculated to each side of nose to cause infection. For control, additional groups of animals were allocated to receive 10 µg or 1 µg of Poly(I:C) alone, an PR8HA vaccine alone, or no treatment. Three days after infection, nasal washings and serum were recovered; IgA in the nasal washings and IgG in the serum were measured using the ELISA method, and the viral titer in the nasal washings was measured by a plaque assay using MDCK cells.

Similarly nasally immunized mice were infected with 20 µl of the virus at a lethal dose of 40 LD₅₀ (10^{4.7} EID₅₀ (about 50000 times the amount of virus showing infectivity to 50% of developed eggs), and their survival was examined.

To evaluate the protective effects on cross infections of nasal influenza vaccines using Poly(I:C) as an adjuvant, vaccines of different subtypes of influenza virus H1N1 (A/PR8) strain, H1N1 (Beijing) strain, H1N1 (A/Yamagata) strain, and H3N2 (A/Guizhou) strain, along with 3 µg of Poly(I:C), were inoculated nasally; three weeks later, each vaccine alone was inoculated; two weeks later, 1.2 µl of 100 pfu of the PR8 influenza virus was inoculated to each side of nose to cause infection. Three days after infection, nasal washings and serum were recovered; IgA in the nasal washings and IgG in the serum were measured using the ELISA method, and the viral titers in the nasal washings were measured by a plaque assay using MDCK cells.

### (Results)

Antibody induction and infection protection with nasal influenza vaccines using Poly(I:C) as an adjuvant

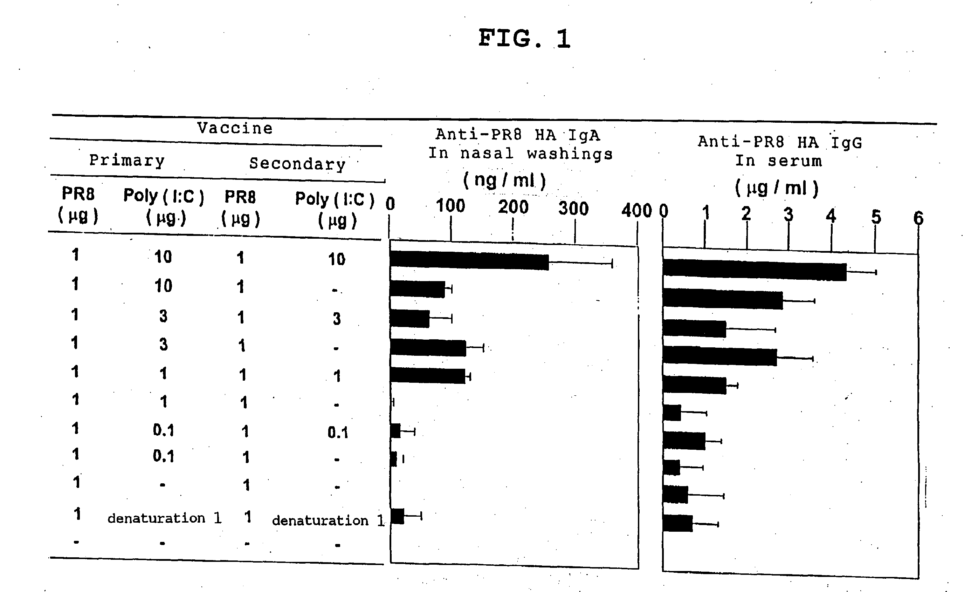

The mucosal adjuvant potential of Poly(I:C) was evaluated. Six weeks previously, 1 µg of each PR8 vaccine, along with a variable amount of 0.1 µg to 10 µg of Poly(I:C), was inoculated nasally; two weeks previously, the same amount of the vaccine, alone or along with an adjuvant, was inoculated nasally. IgA antibody responses in the nasal mucosa and blood IgG responses are shown in Figure 1. To determine the adjuvant effect depending on Poly(I:C) dose, adjuvant action was examined with the amount of Poly(I:C) increased stepwise from 0.1 µg to 10 µg. As a result, IgA responses in the nasal mucosa were observed when a minimum of 0.1 µg of Poly(I:C) was used in the first time of immunization. The amount of IgA induced in the nasal mucosa was dependent on the amount of Poly(I:C); the adjuvant effect of Poly(I:C) was enhanced with the increase in the amount thereof. When 1 µg of Poly(I:C) was used in both times of immunization, 100 ng/ml or more of IgA secretion was observed in nasal washings; when the same was used only in the first time of immunization, 100 ng/ml or more of specific IgA was induced with the addition of 3 µg of Poly(I:C). IgG in serum was determined at the same time, and correlated with IgA secretion; when 1 µg of the PR8 vaccine, along with Poly(I:C), was given for immunization twice at an interval of 4 weeks, a blood IgG level of 1.5 µg/ml was obtained.

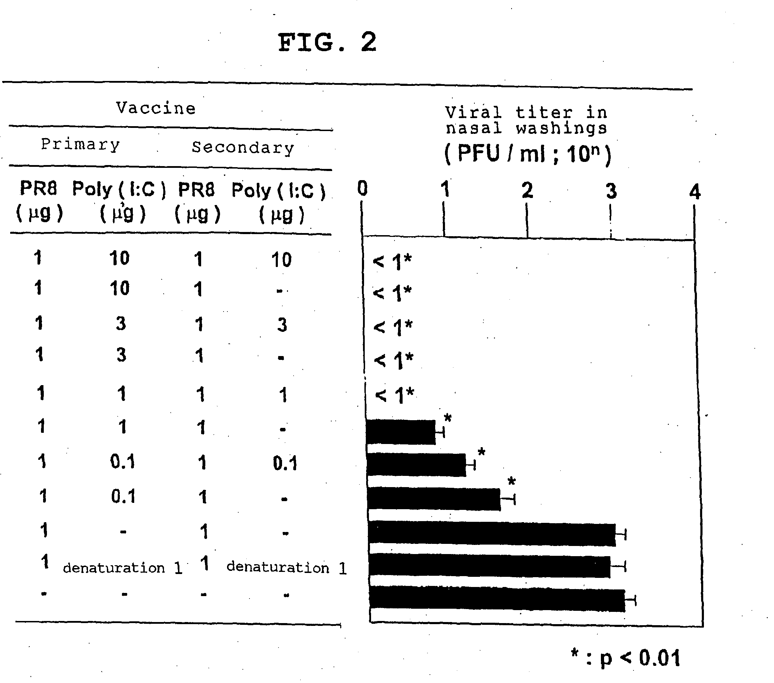

Also, under the same immunization conditions, at 2 weeks after second immunization, 1.2 µl of 100 pfu of the PR8 virus was inoculated to each side of nose to cause infection. In the non-vaccinated control group, viral growth was observed with viral titers of 10³ pfu/ml or more in nasal washings (Figure 2). However, in the group receiving two times of nasal vaccination in combination with Poly(I:C), viral growth was completely suppressed; in the group twice immunized with 1 µg or more of the vaccine alone, and the group receiving 3 µg or more of Poly(I:C) used only at the time of first immunization, absolutely no viral suppressive effect was observed (Figure 2).

Also, even in the groups receiving 1 µg or 0.1 µg of Poly(I:C) used in combination only in the first time of immunization, viral growth was suppressed remarkably to 10^{0.8} pfu/ml and 10^{1.6} pfu/ml, respectively. In the group receiving the vaccine alone twice, absolutely no viral growth suppression was observed.

To demonstrate that a double-stranded RNA structure is important to the adjuvant action of Poly(I:C), Poly(I:C) was heated at 100°C for 5 minutes and immediately cooled on ice; 1 µg was inoculated nasally along with the vaccine. As a result, IgA responses, which were observed at 121 µg/ml without the denaturation, decreased dramatically to 21 µg/ml, and the blood IgG response decreased from 1.5 µg/ml to 0.7 µg/ml.

Also, the viral growth suppressive effect was no longer observed after the denaturation of Poly(I:C).

Next, the protective effect of nasal vaccination in combination with Poly(I:C) against pneumonia due to infection with a lethal dose of influenza virus was examined. Six weeks previously, 1 µg of the PR8 vaccine, in combination with 10 µg, 3 µg, or 1 µg of Poly(I:C), was inoculated nasally; two weeks previously, booster immunization with the vaccine alone was performed; after infection with 20 µl of 40 LD₅₀ of the PR8 virus, the potential for pneumonia prevention was examined. In the non-vaccinated group, 5/5 mice died within 1 week, with the pulmonary viral titer after 3 days being as high as 10⁶ pfu or more. However, in the vaccinated group, all mice survived with combination of 1 µg or more of Poly(I:C). The results are shown below.

**[Table 1]**

| Vaccine | | | | Challenge (40 LD₅₀) | Pulmonary viral titer (PFU/ml;10ⁿ) | Number of surviving mice/test mice |
|---|---|---|---|---|---|---|
| Primary | | Secondary | | | | |
| PR8 (µg) | Poly (I:C) (µg) | PR8 (µg) (µg) | Poly (I:C) (µg) | | | |
| 1 | 10 | 1 | 10 | A/PR8 | <0* | 5/5 |
| 1 | 10 | 1 | - | A/PR8 | N.D. | 5/5 |
| 1 | 3 | 1 | - | A/PR8 | N.D. | 5/5 |
| 1 | 1 | 1 | - | A/PR8 | N.D. | 5/5 |
| 1 | CTB* | 1 | CTB* | A/PR8 | <0* | 5/5 |
| - | - | - | - | A/PR8 | 6.2±5.4 | 0/5 |

As shown above, Poly(I:C) was found to be capable of inducing mucosal IgA antibody responses sufficient to produce protection against the infection as an adjuvant.

### Example 2: Protection against cross infections using nasal vaccines in combination with Poly(I:C)

Regarding protection against influenza viruses induced by nasal influenza vaccines in combination with Poly(I:C), the potentials for protection against cross infections were examined. Each of vaccines of influenza virus strains of subtypes different from PR8, i.e., H1N1 (A/Beijing) strain, H1N1 (Yamagata) strain, and H3N2 (A/Guizhou) strain, along with 3 µg of Poly(I:C), was inoculated for first immunization; four weeks later, the vaccine of the same strain alone was inoculated; two weeks later, animals were infected with 100 pfu of H1N1 (A/PR8) strain; three days later, IgA showing cross reactions with PR8 in nasal washings, and IgG in serum were measured, and protection against cross infections using the PR8 virus was examined.

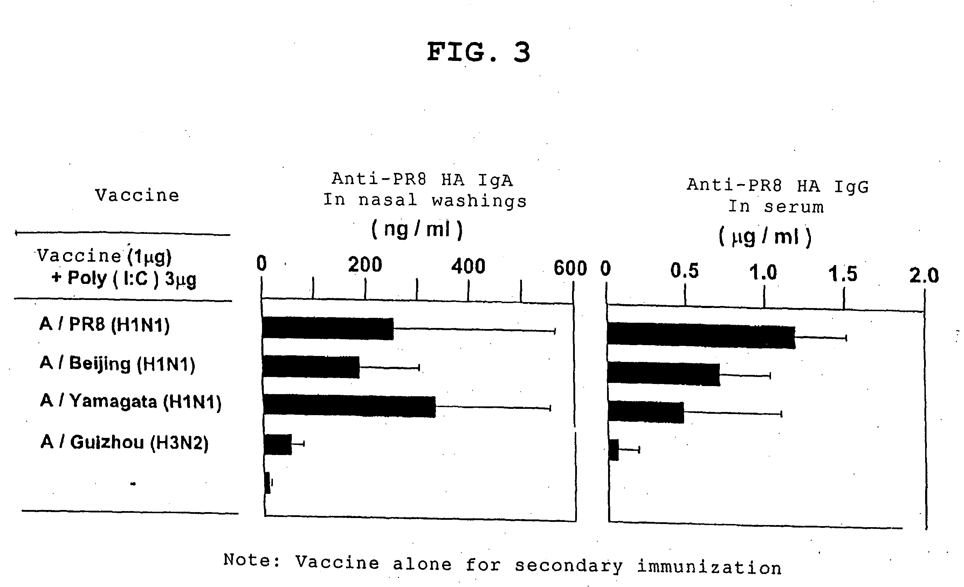

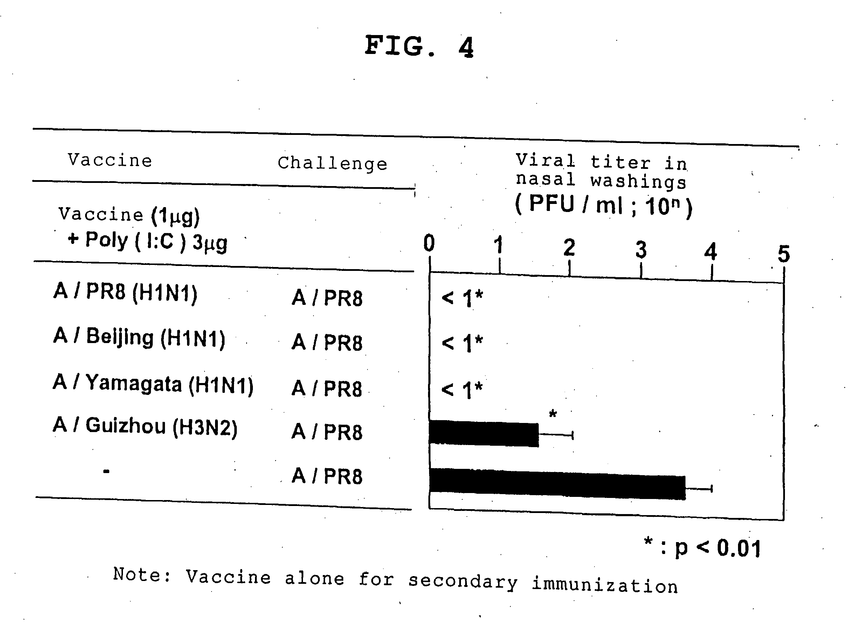

As shown in Figures 3 and 4, both IgA and IgG responses were observed for the H1N1 (A/Beijing) strain and H1N1 (A/Yamagata) strain, which are of the same subtype; viral infection was completely suppressed. For the H3N2 (A/Guizhou) strain, which is of a different subtype, small amounts of IgA and IgG showed cross reactions; partial protection against viral infections was observed.

Hence, protection against cross reactions using nasal influenza vaccines in combination with Poly(I:C) was verified.

### Example 3: Central nervous safety of Poly(I:C)

When Poly(I:C) is used as a nasal vaccine for humans, central nervous safety is important because of the proximity of the nasal cavity to the brain. With this in mind, intracerebral inoculation to BALB/c mice was attempted to verify the safety of Poly(I:C). 0.25 µg, 2.5 µg, or 25 µg of Poly(I:C) was dissolved in 25 µl of PBS, and intracerebral inoculation was performed using a double-needle syringe. After inoculation, body weight changes were measured and survival was checked. For control, 25 µg, 10 µg, or 25 µg of CTB* (CTB comprising 0.1% CT) was dissolved in 25 µl of PBS and intracerebral inoculation was performed in the same manner.

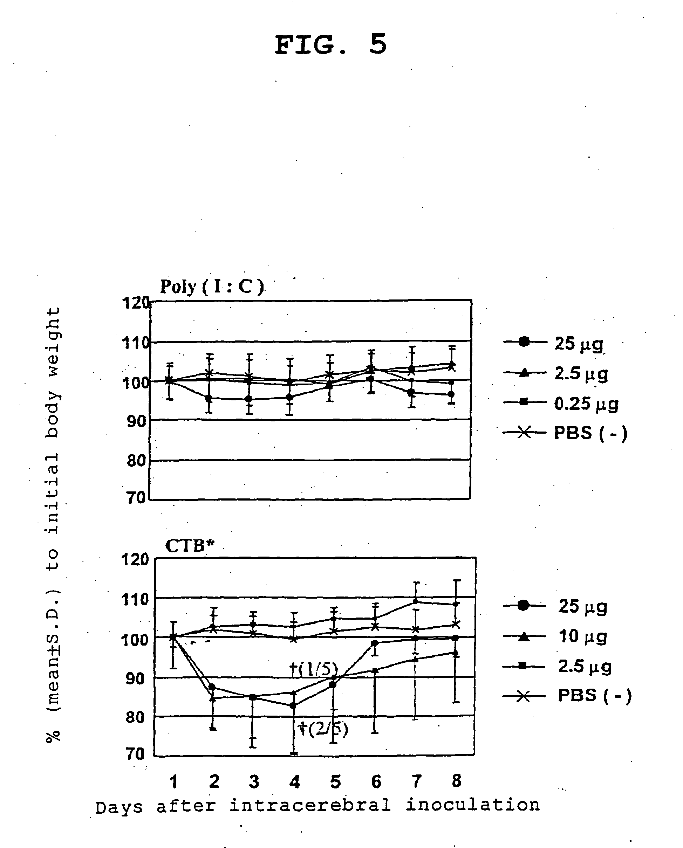

As shown in Figure 5, all mice in all Poly(I:C) intracerebral inoculation groups survived for 2 weeks or more, with only a body weight change of a 5% loss observed in the 25 µg inoculation group. On the other hand, in the groups receiving intracerebral inoculation of control CTB* (CTB with 0.1% CT), 1/5 animals with 10 µg administration and 2/5 animals with 25 µg administration died on day 4, with a body weight loss of as much as 15%.

### (Discussion)

It is evident from a large number of research results that secretory IgA antibodies in the mucosa are more effective in protecting against influenza virus infections than IgG antibodies induced by currently available vaccines. Although nasal vaccines are effective in inducing IgA in the mucosa, no adjuvants usable in humans have yet been established despite many attempts. Poly(I:C), a synthetic double-stranded RNA, has been successfully given to humans by intravenous injection, is effective in inducing IgA, and is considered to be useful for applications to humans as an adjuvant for nasal vaccines.

Judging from the experimental results of this Example, Poly(I:C) is highly useful as an adjuvant for nasal vaccines that are effective in protecting against influenza virus infections in the mucosa. Furthermore, applications to mucosal vaccines for other pathogens are also likely.

In protecting against respiratory infections such as influenza, specific IgA antibodies secreted from the mucosa are highly effective. Protection against cross infections with different types of virus is accounted for mainly by IgA antibodies secreted in the mucosa; humans achieving a recovery from spontaneously caught influenza have such an IgA antibody induced and hence can tolerate infections in the epidemic of the same subtype of mutant viruses. Although vaccination is available as a method of protecting against infections in uninfected individuals, currently available vaccines for subcutaneous inoculation do not produce mucosal immune responses; there is a need for the development of a more effective vaccine having the potential for protection against cross infections. Although a method of nasally inoculating an antigen is available to induce secretory IgA in the mucosa, no sufficient antibody responses are observed with antigen inoculation alone; to achieve more effective immune responses, it is necessary to administer an adjuvant simultaneously with the vaccine.

In this Example, the present inventors demonstrated IgA secretion in the nasal mucosa, IgG responses in serum, and prevention against infections with lethal amounts of influenza viruses, using Poly(I:C), a synthetic double-stranded, as an adjuvant effective in inducing mucosal immunity.

The resulting immune responses included induction of antibodies against viruses of different subtypes from the vaccine strain, and also included protection against infections with viruses of different subtypes from the vaccine strain; a potential for protection against cross infections was verified. Also, in considering human applications of nasal vaccines, adjuvant safety is of concern. Since nasal inoculation, the route of administration used in this Example, involves an administration site very close to the central nervous system, a 0.25 µg to 25 µg/mouse amount of Poly(I:C) was given by intracerebral inoculation to assess its nervous effects and safety. As a result, with control cholera toxin (CTB), which was prepared by adding 0.1% whole toxin to the cholera toxin B subunit, some mice died on day 4 (1/5 in the 10 µg dose group, 2/5 in the 25 µg dose group), with a body weight loss of 15% or more observed, whereas in the Poly(I:C) dose groups, all mice survived for 8 days, with only a body weight loss of about 5% observed transiently in the 25 µg inoculation group; no deaths occurred and safety was verified even with an excess amount of intracerebral inoculation.

Although currently available inactivated influenza HA vaccines are effective vaccines, their potentials for IgA antibody induction in the respiratory mucosal epithelium, which is the door through which the influenza virus enters the body, are low; therefore, improving the potentials are considered to enable further enhancement of the effects of the vaccines. The experiments in this Example showed that virus-specific IgA is efficiently induced on the mucosal surface when currently available inactivated influenza HA vaccines, along with Poly(I:C) added as an adjuvant, are administered nasally. Also, the experiments in mice suggested that fatal infections due to viral challenge are prevented, and that the vaccines are also effective against challenge by other strains of viruses.

Also, regarding applicability, in addition to the influenza virus, Poly(I:C) is likely to be useful as an adjuvant for inactivated antigen vaccines of pathogens that infect via respiratory and other mucosal routes (varicella virus, measles virus, mumps virus, poliovirus, rotavirus, coronavirus, adenovirus, herpes virus, rubella virus, SARS virus, HIV, Bordetella *pertussis, Neisseria meningitidis, Haemophilus influenzae* type b, *Streptococcus pneumoniae* and *Vibrio cholerae* and the like).

### Example 4: Prophylactic effect of inactivated virus particles used as a nasal influenza vaccine in combination with Poly(I:C)

The usefulness of a nasal vaccine in combination with Poly(I:C) was verified not only for the currently available ether-treated HA (Split-product vaccine), but also for another form of vaccine.

### (Materials)

Vaccines: Ether-treated HA vaccine (produced by The Research Foundation for Microbial Diseases of Osaka University), formalin-inactivated whole particle vaccine, A/New Caledonia/20/99 (H1N1) virus) (produced by The Research Foundation for Microbial Diseases of Osaka University)
Mice: BALB/c mice (6 weeks of age, female)

### (Method)

A formalin-inactivated whole particle vaccine of the A/New Caledonia/20/99 (H1N1) virus (0.1 µg), as a vaccine component of a nasal influenza vaccine in combination with Poly(I:C) (100-1000 bp, Sigma) (0.1 µg), was administered to BALB/c mice (6 weeks of age, female); three weeks later, the same vaccine was administered for the second time.

One week later, antibody responses to HA and NA in nasal washings and serum from the mice were measured as indicators of mucosal and systemic protective immunity, respectively.

### (Results)

Also when inactivated whole virus particles were used as a vaccine component of the nasal influenza vaccine in combination with Poly(I:C), mucosal protective immunity and systemic protective immunity were enhanced.

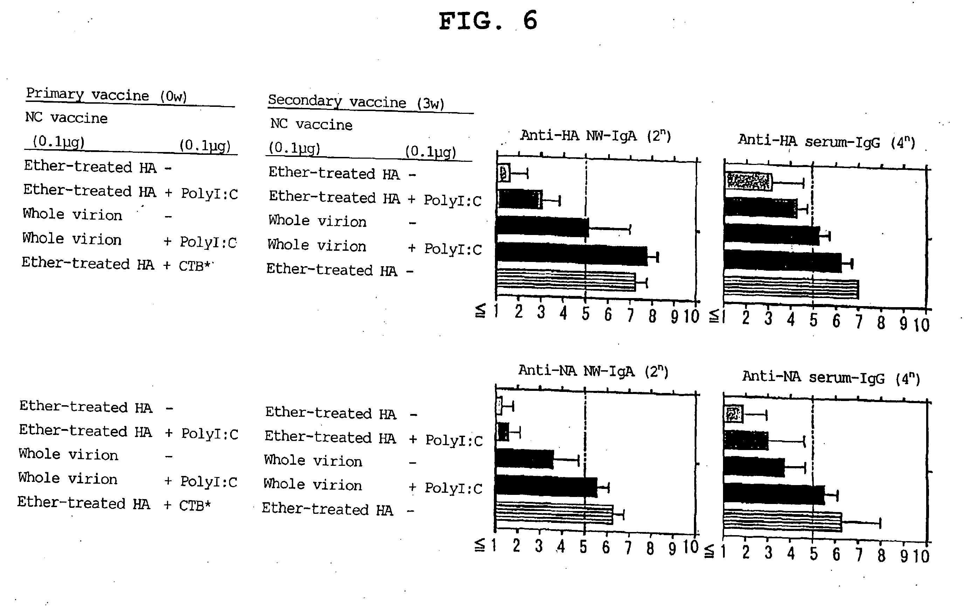

Moreover, even when each of the vaccine and Poly(I:C) was used in an amount of 0.1 µg, the combination produced responses equivalent to those observed in the positive control group of an adjuvant activity expected to provide complete protection against viral infections using the split-product vaccine in combination with CTB*. Furthermore, these responses were higher than those observed with the split-product vaccine used in combination with Poly(I:C). Therefore, it was evident that the nasal vaccine in combination with Poly(I:C) was useful not only when the split-product vaccine was used alone, but also when another form of vaccine was used (Figure 6).

### Example 5: Molecular sizes of Poly(I:C) as an adjuvant for nasal influenza vaccine

Molecular sizes of Poly(I:C) useful for an adjuvant of a nasal influenza vaccine were examined.

### (Materials)

Virus: A/New Caledonia/20/99 (H1N1) virus
Poly(I:C) sizes: (L) 10-300 bp (Fluka), (M) 100-1000 bp (Sigma), (H) >3.3×10⁶ bp (Fluka)
Mice: BALB/c mice (6 weeks of age, female)

### (Method)

A split-product vaccine of the A/New Caledonia/20/99 (H1N1) virus (0.4 µg), along with 0.1 µg of various sizes of Poly(I:C) (10-300 bp (Fluka), (M) 100-1000 bp (Sigma), (H) >3.3x10⁶ bp (Fluka)), was administered nasally to BALB/c mice (6 weeks of age, female); three weeks later, the same vaccine was administered for the second time. One week later, antibody responses to HA and NA in nasal washings and serum from the mice were measured as indicators of mucosal and systemic protective immunity, respectively.

### (Results)

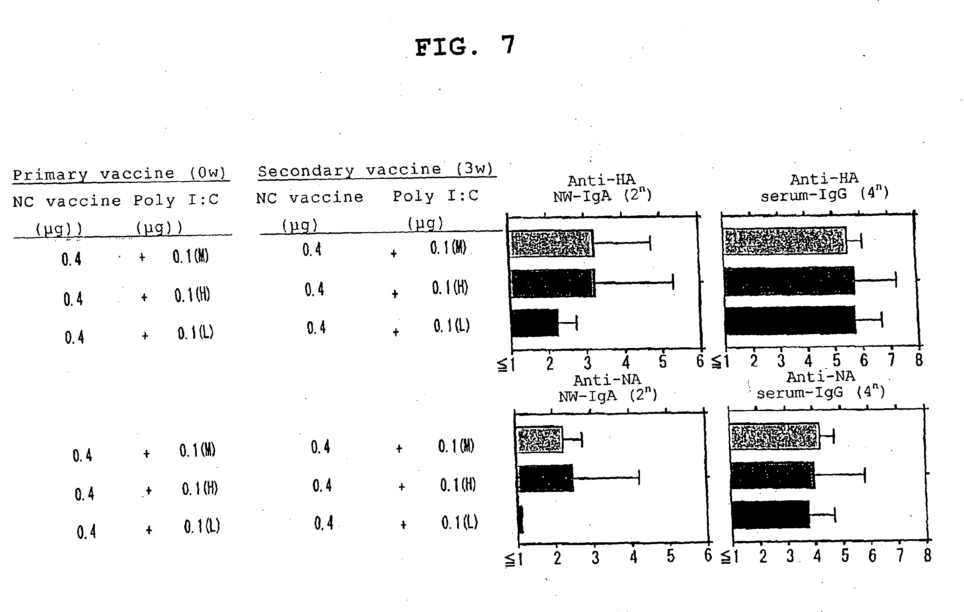

In an experimental group using Poly(I:C) molecular sizes of 10-300 bp, lower mucosal immune responses were observed than the two other groups. Therefore, Poly(I:C) molecular sizes of about 300 bp or more are considered to be useful for an adjuvant for a nasal influenza vaccine (Figure 7).

### Example 6: Adjuvant action of non-Poly(I:C) double-stranded RNA

The action of Poly(I:C) as an adjuvant for nasal influenza vaccines was compared with Poly(A:U), another double-stranded RNA, and Poly(A,U), a single-stranded RNA.

### (Materials)

Subunit: Purified HA
Adjuvants: Poly(I:C), Poly(A:U), and Poly(A,U) .

### (Method)

The HA molecule was purified from the A/New Caledonia/20/99 (H1N1) virus using a column coupled with a specific anti-HA monoclonal antibody, and 1 µg thereof, along with 1 µg of Poly(A:U) (Sigma) or single-stranded Poly(A,U) (Sigma), was administered nasally to BALB/c mice (6 weeks of age, female); three weeks later, HA alone was administered for the second time. One week later, antibody responses to HA in nasal washings and serum from the mice were measured as indicators of mucosal and systemic protective immunity, respectively.

### (Results)

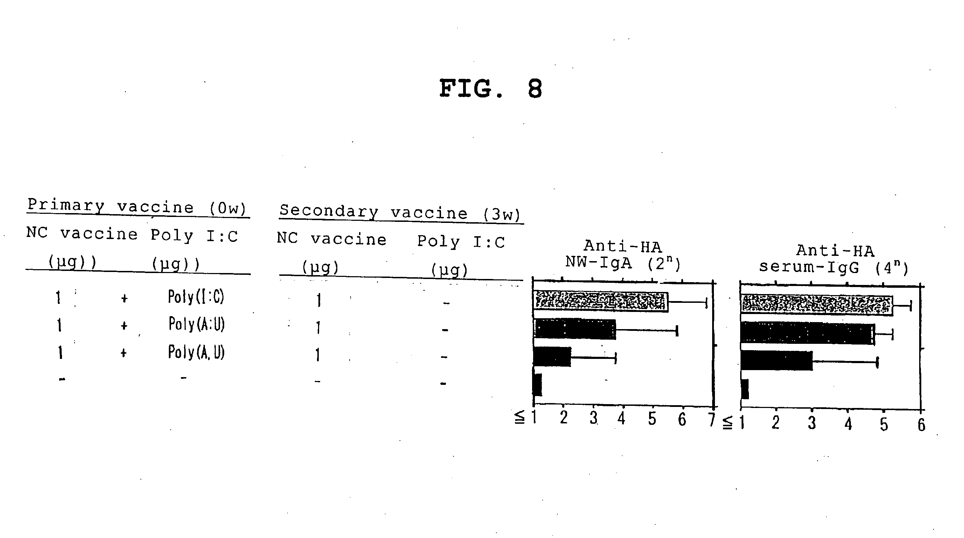

Adjuvant activity was observed also with Poly(A:U) and single-stranded Poly(A,U). Compared to the adjuvant activity of Poly(I:C), Poly(A:U) and single-stranded (A,U) were less active in this order (Figure 8) Hence, it was verified that a non-Poly(I:C) double-stranded RNA also exhibits adjuvant activity when used in combination with a nasal influenza vaccine. Example 7: Induction of protective immunity with some influenza virus subunits under the conditions of nasal administration in combination with Poly(I:C)

It was verified that under the conditions of nasal administration in combination with Poly(I:C), some influenza virus subunits induce protective immunity.

### (Materials)

Subunits: HA, NA, M1 and NP
Adjuvant: Poly(I:C) (100-1000 bp, Sigma)
Mice: BALB/c mice (6 weeks of age, female)

### (Method)

The protective-effect-induction potentials of the influenza virus subunits HA, NA, M1 and NP administered nasally along with Poly(I:C) were compared. Specifically, HA, NA, M1 and NP molecules were purified from the A/NewCaledonia/20/99 (H1N1) virus using a specific anti-monoclonal antibody-conjugated column; a 1 µg portion of each purified product was administered nasally along with 1 µg of Poly(I:C) (100-1000 bp, Sigma) to BALB/c mice (6 weeks of age, female); three weeks later, each molecular species was administered for the second time. One week later, antibody responses to each molecular species in nasal washings and serum from the mice were measured as indicators of mucosal and systemic protective immunity.

### (Results)

Poly(I:C) was shown to enhance mucosal and systemic immune responses to all subunits. However, although the prophylactic effect was increased by enhancing the humoral immunity against HA and NA, the prophylactic effect could not be increased by enhancing the humoral immunity against NP. Hence, it was found that HA and NA are strong protective antigens, and that the potential for induction of prophylactic effect differs among different subunits.

### Example 8: Frequency and interval of administration that increase the efficacy of nasal influenza vaccine in combination with Poly(I:C)

Frequency and interval of administration that increase the efficacy of a nasal influenza vaccine in combination with Poly(I:C) are determined.

### (Materials)

Vaccine: Split-product vaccine of A/New Caledonia/20/99 (H1N1) virus (1 µg)
Adjuvant: Poly(I:C) [100-1000 bp, Sigma]
Mice: BALB/c mice (6 weeks of age, female)

### (Method)

To determine frequency and interval of administration that increase the efficacy of a nasal influenza vaccine in combination with Poly(I:C), a Split-product vaccine of the A/New Caledonia/20/99 (H1N1) virus (1 µg), along with 1 µg of Poly (I : C) (100-1000 bp, Sigma), was administered nasally to BALB/c mice (6 weeks of age, female); 1, 3, 4, and 6 weeks later, the same vaccine was administered for the second time. One or two weeks later, antibody responses to HA and NA in nasal washings and serum from the mice were measured as indicators of mucosal and systemic protective immunity, respectively. Additional experimental groups were allocated to receive only one time of administration and to be examined one and eight weeks later.

### (Results)

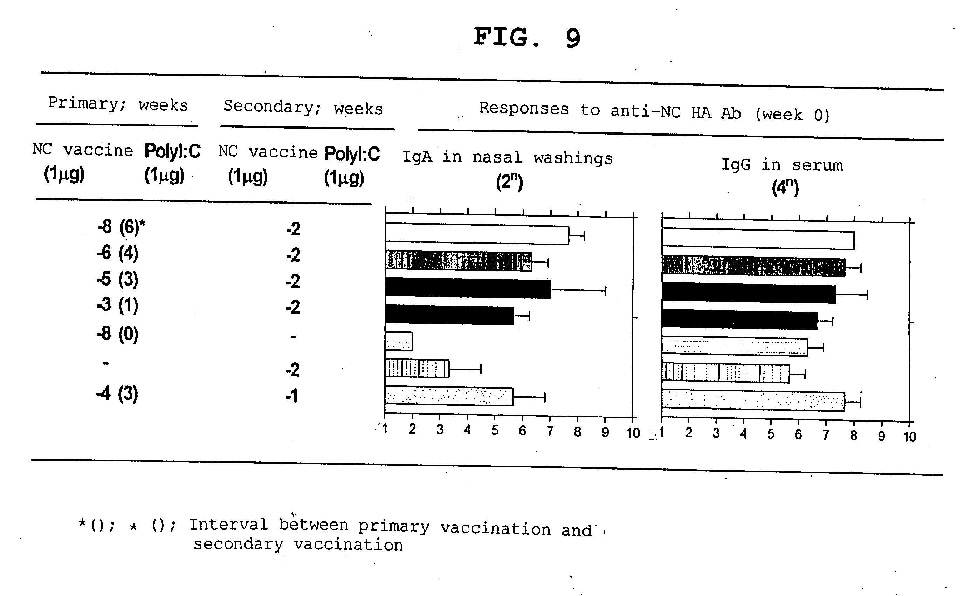

Two times or more of administration at an interval of one week or more was effective in increasing the efficacy of the nasal influenza vaccine in combination with Poly(I:C) (Figure 9).

### Example 9: Prophylactic effect of nasal influenza vaccine in combination with Poly(I:C) in humans

The efficacy of a nasal influenza vaccine in combination with Poly(I:C) in humans is verified on the basis of anti-influenza antibody responses.

### (Materials)

Vaccine: Currently available trivalent influenza vaccine [a split-product vaccine derived from the three viral strains of A/NewCaledonia (H1N1), A/Panama (H3N2), and B/Shangdong] (The Research Foundation for Microbial Diseases of Osaka University)
Adjuvant: Poly(I:C) (100-1000 bp, Sigma)
(Subjects)

Two to several healthy humans

### (Method)

300 µL (150 µL for each of the left and right nasal cavities) of a liquid containing 400 µg/ml of the trivalent vaccine and 700 µg/ml of PolyI:C is administered by spraying to healthy adults; four weeks later, the same liquid is administered again. Two weeks after re-administration, saliva and serum materials are collected and assayed for antibody responses to HA and NA. By comparing the antibody titers before administration and after two times of administration, the antibody response induction potential of this nasal vaccine is evaluated.

### (Results)

In the subjects, increases in IgA antibody against the three strains in the vaccine in saliva are observed in saliva.

Also, in some subjects, increases in anti-NA-IgG antibody and HI antibody in serum are observed.

### Example 10: Enhancement of immune potential by nasal administration of pertussis vaccine along with Poly(I:C)

Enhancement of immune potential was verified when a vaccine for pertussis, a non-influenza infectious disease, was administered nasally along with Poly(I:C).

### (Materials)

Vaccine: Pertussis vaccine (produced by The Research Foundation for Microbial Diseases of Osaka University)
Adjuvant: Poly(I:C) (100-1000 bp, Sigma)
Mice: BALB/c mice (6 weeks of age, female)

### (Method 1)

A pertussis vaccine (produced by The Research Foundation for Microbial Diseases of Osaka University) (1 to 3 µg), along with 0.1 µg to 10 µg of Poly(I:C) (100 to 1000 bp, Sigma), was administered nasally to BALB/c mice (6 weeks of age, female); three weeks later, the same vaccine was administered for the second time. One week after second administration, antibody responses to the pertussis vaccine in nasal washings and serum from the mice were measured by the ELISA method, and used as indicators of mucosal and systemic protective immunity.

### (Results)

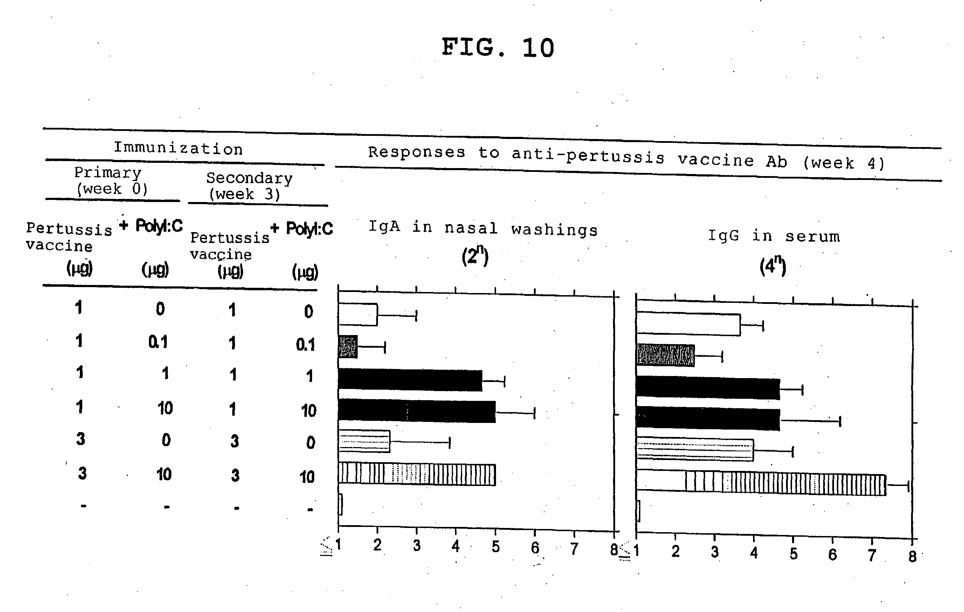

Poly(I:C) also increased protective immunity against the pertussis vaccine and suggested to be also effective in enhancing protective immunity for vaccines against non-influenza infections (Figure 10).

### (Method 2)

A pertussis vaccine (produced by The Research Foundation for Microbial Diseases of Osaka University) (1 to 3 µg), along with 0.1 to 10 µg of Poly(I:C) (100-1000 bp, Sigma), was administered nasally to BALB/c mice (6 weeks of age, female); three weeks later, the same vaccine was administered for the second time. One week after second administration, antibody responses to the pertussis vaccine in nasal washings and serum from the mice were measured by the ELISA method, and used as indicators of mucosal and systemic protective immunity. Two to three weeks after second administration, a virulent strain of Bordetella *pertussis* was inoculated into the brain or nasal cavity (spraying) of each immunized mice, and the animals were observed for 14 days; effects were estimated from the survival rates of the immunized mice.

### (Results)

Judging from the mouse survival rates, it was verified that Poly(I:C) also increases protective immunity against the pertussis vaccine, and is also effective in enhancing protective immunity for vaccines against non-influenza infectious diseases.

### Example 11: Prophylactic effect of varicella vaccine administered nasally along with Poly(I:C) in humans

Regarding the safety and efficacy of a nasal live varicella vaccine in combination with Poly(I:C) given by booster inoculation in adults, nasal mucosal inoculation is performed, and humoral immunity and cellular immunity are compared with a group receiving nasal inoculation of the varicella vaccine alone.

### (Materials)

Vaccine: Live varicella vaccine (produced by The Research Foundation for Microbial Diseases of Osaka University)
Adjuvant: Poly(I:C) (100-1000 bp, Sigma)
Healthy humans: Two to several subjects per group

### (Method)

Physiological saline for injection is added to two vials/human of the live varicella vaccine, and this is inoculated nasally to healthy adults using a nebulizer. Also, 300 µl (150 µl for each of the left and right nasal cavities) of a vaccine liquid containing a currently available vaccine and Poly(I:C) (100-1000 bp, Sigma) is administered by spraying. By measuring humoral immunity and cellular immunity, prophylactic effects are verified.

### (Results)

Poly(I:C) also increases protective immunity for the live varicella vaccine, and is suggested to be also effective in enhancing protective immunity for vaccines against non-influenza infectious diseases.

Although the present invention has been exemplified by means of preferred modes of embodiment of the invention above, it is understood that the scope of the present invention is to be limited only by the Scope of Claims. The patents, patent applications and documents cited herein are understood to be such that the teachings thereof should be referenced for the present description as specifically described herein.

### Industrial Applicability

The present invention provides a form of vaccine that enables easy vaccination by mucosal administration and obtainment of cross immunity. In measures against influenza viruses, for example, it is thereby possible to produce an effective vaccine, which vaccine is likely to be used widely to take efficient prophylactic measures in pharmaceutical and other industries.

## Claims

1. A vaccine for mucosal administration comprising:
A) a double-stranded RNA; and
B) a subunit antigen or inactivated antigen of a pathogen.

2. The vaccine of claim 1, wherein said mucosa comprises the nasal mucosa.

3. The vaccine of claim 1, wherein said pathogen is selected from the group consisting of varicella virus, measles virus, mumps virus, poliovirus, rotavirus, influenza virus, adenovirus, herpes virus, rubella virus, severe acute respiratory syndrome virus (SARS virus), human immunodeficiency virus (HIV), Bordetella *pertussis, Neisseria menin gitidis, Haemophilus influenzae* type b, *Streptococcus pneumoniae* and *Vibrio cholerae.*

4. The vaccine of claim 1, wherein said pathogen is an influenza virus.

5. The vaccine of claim 1, wherein said subunit comprises at least one subunit selected from the group consisting of the influenza virus subunits HA, NA, M1, M2, NP, PB1, PB2, PA and NS2.

6. The vaccine of claim 1, wherein said double-stranded RNA is present at a concentration sufficient to produce secretory IgA.

7. The vaccine of claim 1, wherein said double-stranded RNA is present at a concentration of 0.1 to 10 mg/ml.

8. The vaccine of claim 1, wherein the size of said double-stranded RNA is 10²-10⁸ bp.

9. The vaccine of claim 1, wherein said subunit comprises at least NA or HA.

10. The vaccine of claim 1, wherein said double stranded RNA comprises Poly(I:C).

11. A method of preventing an infectious disease, comprising a step for mucosally administering at least once:
A) a vaccine for mucosal administration comprising:
a) a double-stranded RNA; and
b) a subunit antigen or inactivated antigen of a pathogen.

12. The method of claim 11, wherein said vaccine is administered at least twice.

13. The method of claim 11, wherein said vaccine is administered at an interval of at least 1 week or more.

14. The method of claim 11, wherein said double-stranded RNA comprises Poly(I:C).

15. A vaccine kit for preventing an infectious disease, provided with:
A) a vaccine for mucosal administration comprising:
a) a double-stranded RNA; and
b) a subunit antigen or inactivated antigen of a pathogen; and
B) an instruction sheet directing to mucosally administer said vaccine at least once.

16. The kit of claim 15, wherein the aforementioned vaccine is administered at least twice.

17. The kit of claim 15, wherein said vaccine is administered at an interval of at least 1 week or more.

18. The kit of claim 15, wherein said double-stranded RNA comprises Poly (I : C) .

19. A use of a double-stranded RNA for mucosal administration of a vaccine.

20. The use of claim 19, wherein said double-stranded RNA comprises Poly(I:C).

21. A use of a double-stranded RNA for production of a vaccine for mucosal administration.

22. The use of claim 21, wherein said double-stranded RNA comprises Poly(I:C).
